# DEMANDE DE BREVET EUROPEEN

(11) **EP 1 547 578 A2**
(43) Date de publication de la demande: **29.06.2005**
(21) Numéro de dépôt: 04293077.6
(22) Date de dépôt: 22.12.2004
(51) Int. Cl.: A61K 7/48

(54) **Utilisation d'un ligand stéroidien ou non-stéroidien du récepteur EcR dans une préparation cosmétique ou dermatologique pour maintenir l'homéostasie cutanée**

(30) Priorité: 22.12.2003 FR 0315156
(71) Demandeur: L'OREAL, 75008 Paris (FR)
(72) Inventeur: Ferraris, Corinne, 75017 Paris (FR); Rathman-Josserand, michelle, 78170 La-Celle-Saint-Cloud (FR); Castiel, Isabelle, 06000 Nice (FR)
(74) Mandataire: Dossmann, Gérard

(57) **Abrégé**

La présente invention a pour objet l'utilisation d'au moins un ligand du récepteur EcR dans une préparation cosmétique ou dermatologique destinée à maintenir l'homéostasie cutanée.

La présente invention a aussi pour objet un procédé de traitement cosmétique pour maintenir l'homéostasie cutanée consistant en l'application d'une composition selon l'invention.

## Description

La présente invention a pour objet l'utilisation d'au moins un ligand du récepteur EcR dans une préparation cosmétique ou dermatologique destinée à maintenir l'homéostasie cutanée.

La présente invention a aussi pour objet un procédé de traitement cosmétique pour maintenir l'homéostasie cutanée consistant en l'application d'une composition selon l'invention.

L'homéostasie épidermique est définie comme étant le maintien de l'épiderme dans un état physiologique c'est-à-dire que le renouvellement et la différenciation des cellules épidermiques doivent être préservés dans un état d'équilibre.

En effet, l'épiderme est un épithélium pluristratifié kératinisé en perpétuel renouvellement. Afin d'assurer une épaisseur constante de l'épiderme, le compartiment germinatif (assise basale) produit un nombre de cellules égal au nombre de celles qui sont éliminées en surface. Cet équilibre fragile est modifié dans le cas de situations pathologiques comme le psoriasis. L'épaisseur épidermique est augmentée et les protéines MRP8/MRP14 marqueurs signant un état de stress et/ou d'hyperprolifération sont alors fortement exprimés. L'épiderme peut également présenter des signes d'atrophie quand le taux de renouvellement cellulaire n'égale pas le taux d'élimination des cellules à la surface.

De manière inattendue, la demanderesse a découvert que l'utilisation des ecdystéroïdes permettait de maintenir l'homéostasie cutanée.

Les ecdystéroïdes sont connus pour jouer un rôle important dans le règne animal aussi bien chez les insectes que dans le règne végétal. Chez les insectes en particulier, ces hormones stéroïdes jouent un rôle clé dans la croissance et la reproduction.

Les ecdystéroïdes dont fait partie l'ecdystérone ou β-ecdysone (2b,3b,5b,22*R*)-2,3,14,20,22,25-hexahydroxycholest-7-en-6-one) ont ainsi été utilisées pour lutter contre les signes de vieillissement cutané et pour améliorer la fonction barrière de la peau. Le brevet US 5198225 décrit en effet l'utilisation en cosmétique des propriétés de régénération de structure cutanée des ecdystéroïdes pour des produits à visée anti-âge et en particulier anti-rides. Le brevet FR 2696075 décrit l'utilisation d'ecdystéroïdes dans des préparations cosmétiques afin de restaurer, préserver et/ou renforcer la fonction protectrice de l'épiderme. Or les ecdystéroïdes n'ont jamais été utilisés pour maintenir l'homéostasie épidermique.

La cible biologique de l'ecdystérone dans la peau est inconnue. L'activité de l'ecdystérone sur la mue et sur la métamorphose chez les arthropodes dépend de l'interaction de cette hormone avec son récepteur EcR. À ce jour, aucun récepteur EcR n'a été décrit chez les mammifères. Pour identifier un tel récepteur, la demanderesse a réalisé un immunomarquage sur coupes histologiques de peau humaine reconstruite avec un anticorps spécifique de l'EcR chez plusieurs insectes (anticorps 9B9, Developmental Biology, 1996, 180 : 258-272). L'analyse des coupes a mis en évidence un marquage très présent autour des noyaux dans la couche basale de la peau ainsi que dans les couches supérieures de l'épiderme et dans le derme.

Un premier objet de l'invention est ainsi l'utilisation dans une composition cosmétique ou dermatologique de ligands du récepteur EcR pour maintenir l'homéostasie cutanée.

Un autre objet de l'invention est un procédé de traitement cosmétique à base de cette composition visant à maintenir l'homéostasie cutanée.

D'autres objets apparaîtront à la lumière de la description et des exemples qui suivent.

Par homéostasie cutanée, on entend maintenir la physiologie de l'épiderme c'est-à-dire préserver le taux normal de renouvellement et de différenciation cellulaire.

L'homéostasie cutanée se traduit ainsi par un maintien de la peau dans des conditions physiologiques de normalité à l'équilibre sans expression de marqueurs liés à l'hyperprolifération ou liés au stress.

Dans le cas d'une atrophie de l'épiderme, le renouvellement cellulaire ne compense plus la perte de cellules éliminées en surface. En travaillant sur un modèle de peau reconstruite *in vitro,* la demanderesse a montré que la présence d'un ligand du récepteur EcR dans le milieu de culture entraîne une augmentation importante de l'épaisseur des couches vivantes de l'épiderme reconstruit sans pour autant diminuer les couches cornées et prévient ainsi l'atrophie de l'épiderme. D'autre part l'augmentation de l'épaisseur épidermique n'est pas liée à une hyperprolifération de cellules. L'un des objets de l'invention est donc de maintenir la peau à une épaisseur adaptée à une expression optimale de ses fonctions, correspondant à l'épaisseur d'un épiderme sain.

Les marqueurs protéiques exprimés dans les processus d'hyperprolifération et/ou de stress (ultraviolets, stress mécanique ou chimique) comprennent les protéines MRP8 et MRP14. La présence détectée de ces protéines dans les cellules de la peau représente donc la signature d'un état physiologique anormal. En effet, on peut détecter la protéine MRP8 dans les épidermes hyperplasiques et dans la peau reconstruite, mais cette protéine n'est jamais retrouvée dans l'épiderme interfolliculaire de la peau normale.

Or, l'addition d'ecdystérone dans le milieu de culture diminue fortement l'expression des gènes codant pour ces protéines ainsi que les protéines elles-mêmes, ce qui signe le retour à une situation physiologique normale de ces cellules.

Différents ligands du récepteur EcR peuvent être utilisés. De préférence, on peut utiliser les ecdystéroïdes (dont l'ecdystérone), l'halofénozide (RH-0345), le méthoxyfénoside (RH-2485), le tébufénozide (RH-5992), les hydrazides (Rohm et Haas Annu. Rev. Entomol. 1998, 43, 545-69), le benzamide (BTBHIB de Sumitomo Chemical Co. (Agricultural Chemical Research Laboratory, Biochemical and Biophysical research Communications 1996, 227, 427-32), la quercétine ou le coumestrol (qui sont tous deux des flavonoïdes oestrogéniques).

La quantité en poids du ou des ligands du récepteur EcR est comprise entre 0,001 % et 10 % et de préférence de 0,01 % à 5 % par rapport au poids final de la composition finale utilisée.

La composition utilisée peut comprendre en plus un ou des composés supplémentaires choisis parmi un agent desquamant, un agent hydratant, un agent dépigmentant ou propigmentant.

La composition selon l'invention peut comprendre en outre de l'eau, et/ou un ou des solvants organiques cosmétiquement acceptables. Ces solvants peuvent être choisis dans le groupe constitué par les solvants organiques hydrophiles, les solvants organiques lipophiles, les solvants amphiphiles ou leurs mélanges,

Parmi les solvants organiques hydrophiles, on peut citer par exemple des mono-alcools inférieurs, linéaires ou ramifiés, ayant de 1 à 8 atomes de carbone comme l'éthanol, le propanol, le butanol, l'isopropanol, l'isobutanol, des polyéthylèneglycols éventuellement oxyéthylénés, des polyols tels que le propylèneglycol, l'isoprèneglycol, le butylène glycol, le glycérol, le sorbitol et ses dérivés, les éthers de glycol et les éthers de propylène glycol.

Comme solvants organiques amphiphiles, on peut citer des polyols tels que des dérivés de propylèneglycol.

Comme solvants organiques lipophiles, on peut citer par exemple les esters gras.

La composition selon l'invention peut également comporter des adjuvants tels que des corps gras, des agents conservateurs, des agents stabilisants, des agents opacifiants, des adoucissants, des silicones, des agents moussants, des agents antioxydants, des agents régulateurs de pH, des agents émulsifiants, des gélifiants et/ou épaississants classiques hydropholes ou lipophiles, des actifs hydropholes ou lipophiles, des parfums, des émulsionnants, des séquestrants, des polymères, des agents acidifiants ou alcalinisants, des charges, des agents anti-radicaux libres, des céramides, des agents hydratants, des vitamines, des tensio-actifs, des antipelliculaires, des propulseurs, des colorants, ou tout autre adjuvant habituellement utilisé en cosmétique.

Les quantités de ces différents adjuvants sont celles classiquement utilisées dans le domaine considéré. L'homme de l'art veillera à choisir le ou les éventuels composés à ajouter à la composition selon l'invention de manière à ne pas ou substantiellement pas altérer les propriétés avantageuses attachées à la composition conforme à l'invention.

Les compositions selon l'invention sont particulièrement appropriées sur le plan cosmétique et/ou dermatologique et ne provoquent pas d'irritation du cuir chevelu, même après un contact prolongé sans rinçage.

La composition selon l'invention comprenant au moins un ligand du récepteur EcR peut se présenter sous la forme d'un gel, d'un lait, d'une lotion, d'un sérum, d'un masque ou d'une crème.

Un autre objet de l'invention est un procédé de traitement cosmétique pour maintenir l'homéostasie cutanée par l'application sur la peau d'une personne, d'une quantité cosmétiquement efficace d'une composition contenant au moins un ligand du récepteur EcR.

Les exemples suivants illustrent l'invention sans toutefois la limiter :

### Exemple 1 :Mesure de l'épaisseur de l'épiderme

Des épidermes reconstruits ont été réalisés suivant la technique de Asselineau et al., 1985 (Asselineau D, Bernard BA, Bailly C. & Darmon M :Epidermal morphogenesis and induction of 67 kD Keratin polypeptide by culture at the air liquid interface *Exp Cell Res* **159 :** 536-539).

Dès le premier jour d'exposition à l'interface air/liquide, les épidermes ont été cultivés en présence d'ecdystérone dans le milieu de culture (1 à 1000 µg/ml). Les épidermes ont été cultivés durant 7 jours. Le traitement a été renouvelé toutes les 48H en changeant le milieu de culture. Après 7 jours de traitement, les épidermes ont été prélevés. L'analyse histologique est réalisée après une coloration HES (Hématoxyline Eosine Safran).

Des mesures d'épaisseur d'épiderme ont été réalisées sur coupes histologiques. Pour cela 10 mesures sont effectuées sur un champ. Pour chaque échantillon, 5 champs sont comptés. Ces mesures sont réalisées à l'aide d'un système d'analyse d'image Leica Q600S avec un objectif X10. L'épaisseur moyenne obtenue pour chaque échantillon correspond à 50 mesures effectuées. L'ensemble des valeurs obtenues est résumé dans le tableau I. :

**Tableau I. :**

| Epaisseur de l'épiderme en µm après 7 jours de traitement par l'ecdystérone. | | |
|---|---|---|
| Témoin | Ecdystérone 1000µg/ml | Ecdystérone 10µg/ml |
| 60.21 (9.04) | 72.05 (16) | 115 (24.07) |

Les chiffres entre parenthèses représentent les écarts type.

L'application de l'ecdystérone dans le milieu de culture des épidermes reconstruits entraîne une augmentation de l'épaisseur de l'épiderme au niveau des couches vivantes. L'augmentation de l'épaisseur de l'épiderme ne s'accompagne pas d'une parakératose (noyau dans la couche cornée) ni d'une diminution de l'épaisseur de la couche cornée. La différenciation de l'épiderme est marquée par la présence de nombreuses couches granuleuses.

### Exemple 2 : Mesures de la multiplication cellulaire en fonction de la quantité d'ecdystérone dans le milieu.

Les kératinocytes proviennent d'une plastie mammaire. Ces kératinocytes ont été isolés puis cultivés en milieu KGM (Clonetics) supplémenté en facteurs de croissance (EGF (Epidermial Growth Factor) : 10ng/ml, 0,4µg/ml, insuline 5µg/ml, BPE (Bovine Pituitary Extract) 4ml/L).

Les cellules sont traitées par l'ecdystérone de 8H à 48H. Il n'y a pas d'EGF dans le milieu de culture pendant le traitement par l'ecdystérone.

L'ecdystérone a été testée aux concentrations suivantes : 1 µg/ml, 0,1 µg/ml et 0,01 µg/ml.

| Concentration d'ecdystérone dans le milieu (en µg/ml) | Temps de traitement (en heures) | Nb de cellules/Flask |
|---|---|---|
| 0 | 8 | 0.8 10⁶ |
| 0.01 | 8 | Problème |
| 0.1 | 8 | 0.8 10⁶ |
| 1 | 8 | 0.7 10⁶ |
| 0 | 16 | 0.8 10⁶ |
| 0.01 | 16 | 0.8 10⁶ |
| 0.1 | 16 | 1.0 10⁶ |
| 1 | 16 | 0.5 10⁶ |
| 0 | 24 | 1.1 10⁶ |
| 0.01 | 24 | 1.0 10⁶ |
| 0.1 | 24 | 1.1 10⁶ |
| 1 | 24 | 1.1 10⁶ |

| Concentration d'ecdystérone dans le milieu (en µg/ml) | Temps de traitement (en heures) | Nb de cellules/Flask |
|---|---|---|
| 0 | 16 | 4,4. 10⁵ |
| 0.001 | 16 | 3.6. 10⁵ |
| 0.01 | 16 | 2,5. 10⁵ |
| 0.1 | 16 | 3,6. 10⁵ |
| 0 | 24 | 5,2. 10⁵ |
| 0.001 | 24 | 3,6. 10⁵ |
| 0.01 | 24 | 5,7. 10⁵ |
| 0.1 | 24 | 5,2. 10⁵ |
| 0 | 48 | 1,9. 10⁶ |
| 0.001 | 48 | 1.6 10⁶ |
| 0.01 | 48 | 1,6. 10⁶ |
| 0.1 | 48 | 1,5. 10⁶ |

Après 48 h de traitement par l'ecdystérone, on n'observe pas d'augmentation significative du nombre de cellules par rapport aux cellules témoins.

L'augmentation de l'épaisseur des couches vivantes de l'épiderme suite à un traitement par l'ecdystérone n'est donc pas liée à une hyperprolifération.

### Exemple 3 : Analyse du cycle cellulaire

L'analyse du cycle cellulaire consiste à étudier le contenu en ADN d'une population cellulaire en cytométrie de flux. Ces études sont réalisées sur des kératinocytes humains normaux cultivés en monocouche dans un milieu défini (KGM - Clonetics).

Les kératinocytes proviennent d'une plastie mammaire. Ces kératinocytes ont été isolés puis cultivés en milieu KGM supplémenté en facteurs de croissance (EGF : 10ng/ml, 0.4 µg/ml, insuline 5µg/ml, BPE (Bovine Pituitary Extract ) 4ml/L).

Les cellules sont traitées par l'ecdystérone durant 8 h à 48 H. Il n'y a pas d'EGF dans le milieu de culture pendant le traitement par l'ecdystérone.

L'ecdystérone a été testée aux concentrations suivantes : 1µg/ml, 0.1µg/ml et 0.01µg/ml.

Le protocole suivi pour l'analyse du cycle cellulaire en cytométrie de flux est décrit dans la publication suivante : Staiano-Coico L., Higgins P.J., Darzynkiewicz Z. *et al*; 1986 Human keratinocyte culture. Identification and stagind of epidermal cell subpopulations. *J. lin. Invest*. **77**, 396-404. L'analyse a été réalisée à l'aide d'un cytomètre de flux Facs Calibur 3C Trieur de la société Becton Dickinson.

L'analyse des différentes phases du cycle cellulaire par comptage en cytométrie de flux révèle une modification de la population cellulaire entre les phases GO/G1 et G2/M en fonction de la quantité d'ecdystérone présent dans le milieu de culture des kératinocytes.

Ainsi, l'augmentation de l'épaisseur des couches vivantes de l'épiderme suite à un traitement par l'ecdystérone est le résultat d'une modification du cycle cellulaire.

### Exemple 4 : Modulation de l'expression de marqueurs impliqués dans les phénomènes hyperprolifératifs : Résultats génomigues

L'ecdystérone a été appliquée dans le milieu des épidermes reconstruits dans les mêmes conditions que l'exemple 1.

Après 7 jours de traitement, les épidermes ont été prélevés. L'analyse de l'expression des ARN messagers (ARNm) montre que le traitement par l'ecdystérone diminue l'expression des ARNm de MRP8/MRP14 d'un facteur 1.7 et de ceux de la psoriasine, qui est un marqueur associé à des états hyperprolifératif et/ou de stress, d'un facteur 3,5.

Ainsi, l'ecdystérone normalise l'expression de marqueurs associés à des états hyperprolifératifs et/ou de stress de l'épiderme.

### Exemple 5 : Modulation de l'expression de marqueurs impliqués dans les phénomènes hyperprolifératifs : Résultats expression des protéines

L'ecdystérone a été ajoutée dans le milieu des épidermes reconstruits dans les mêmes conditions que dans l'exemple 1.

Les protéines MRP8 et MRP14 sont des protéines fortement exprimées dans la peau reconstruite, dans la peau psoriasique ainsi que par les kératinocytes hyperprolifératifs mais ayant cependant gardé une capacité à se différencier. L'analyse des marqueurs protéiques MRP8 et MRP14 a été réalisée par immunofluorescence sur coupe histologique.

L'analyse des coupes histologiques montre que l'ecdystérone restreint l'expression de MRP8 et MRP14 aux couches supérieures de l'épiderme.

### Exemple 6 : Composition cosmétique

L'émulsion multiple est préparée de la manière suivante :
On prépare l'émulsion primaire en mélangeant les constituants de la phase A à température ambiante, en mélangeant par ailleurs les constituants de la phase B à température ambiante et en versant lentement la phase B dans la phase A sous agitation rapide. Pour préparer l'émulsion triple, on prépare les différentes phases, puis on verse lentement la phase A dans la phase B sous agitation rapide. On y ajoute la phase C puis la phase D. On agite jusqu'à homogénéisation complète.

| 1. Emulsion primaire : | |
|---|---|
| Phase A : | |
| - Abil WE09 | 2,5% |
| - Huile de silicone volatile | 17,5% |
| - Polydiméthylsiloxane | 4% |

| Phase B | |
|---|---|
| - Glycérine | 45% |
| - Conservateur | 0,8% |
| - Ecdysterone | 0,2% |
| - Eau déminéralisée | qsp 100% |

| 2. Emulsion multiple : | |
|---|---|
| Phase A : | |
| - Emulsion primaire | 20% |
| - Huile de silicone volatile | 10% |

| Phase B : | |
|---|---|
| - Poly-(acide 2-acrylamido 2-méthylpropane sulfonique) réticulé et neutralisé par de l'ammoniaque | 0,5% |
| - Copolymère acrylate/C₁₀-C₃₀-alkylacrylate (Pemulen TR1) | 0,3% |
| - Conservateurs | 1 % |
| - Eau déminéralisée | qsp % |

| Phase C : | |
|---|---|
| - Triéthanolamine | 0,3% |
| - Eau déminéralisée | 2% |

| Phase D | |
|---|---|
| - Poly-(acide 2-acrylamido 2-méthylpropane sulfonique) réticulé et neutralisé par de l'ammoniaque | 1,5% |
| - Eau déminéralisée | |

## Revendications

1. Utilisation d'au moins un ligand du récepteur EcR pour la préparation d'une composition cosmétique ou dermatologique destinée à maintenir l'homéostasie cutanée.

2. Utilisation selon la revendication 1, **caractérisée en ce que** le ligand du récepteur EcR est un ecdystéroïde.

3. Utilisation selon la revendication 1, **caractérisée en ce que** le ligand du récepteur EcR est l'halofénozide.

4. Utilisation selon la revendication 1, **caractérisée en ce que** le ligand du récepteur EcR est le méthoxyfénoside.

5. Utilisation selon la revendication 1, **caractérisée en ce que** le ligand du récepteur EcR est le tébufénozide.

6. Utilisation selon la revendication 1, **caractérisée en ce que** le ligand du récepteur EcR est un hydrazide.

7. Utilisation selon la revendication 1, **caractérisée en ce que** le ligand du récepteur EcR est le benzamide.

8. Utilisation selon la revendication 1, **caractérisée en ce que** le ligand du récepteur EcR est la quercétine.

9. Utilisation selon la revendication 1, **caractérisée en ce que** le ligand du récepteur EcR est le coumestrol.

10. Utilisation selon l'une des revendications 1 à 9, **caractérisée en ce que** la quantité en poids du ou des ligands du récepteur EcR est comprise entre 0,001 et 10 % par rapport au poids total de la composition finale.

11. Utilisation selon l'une des revendications 1 à 9, **caractérisée en ce que** la quantité en poids du ou des ligands du récepteur EcR est comprise entre 0,01 et 5 % par rapport au poids total de la composition finale.

12. Utilisation selon l'une quelconque des revendications 1 à 11 **caractérisée en ce que** la composition comprend de plus un ou des composés supplémentaires choisis parmi un agent desquamant, un agent hydratant, un agent dépigmentant ou un agent propigmentant.

13. Utilisation selon l'une des revendications 1 à 12, **caractérisée en ce que** la composition comprend en plus, de l'eau, de l'alcool et un ou des solvants organiques cosmétiquement acceptables.

14. Utilisation selon l'une des revendications 1 à 13, **caractérisée par le fait que** la composition comprend en plus, un ou des adjuvants cosmétiquement acceptables.

15. Utilisation selon l'une des revendications 1 à 14, **caractérisée par le fait que** la composition se présente sous la forme d'une solution aqueuse, hydroalcoolique ou d'un gel aqueux ou hydroalcoolique.

16. Utilisation selon l'une des revendications 1 à 15, **caractérisée par le fait que** la composition se présente sous la forme d'un lait, d'un gel, d'une mousse, d'un sérum ou d'une lotion.

17. Procédé de traitement cosmétique pour maintenir l'homéostasie cutanée, **caractérisé en ce qu'**on applique sur la peau d'une personne, une quantité cosmétiquement efficace d'une composition contenant au moins un ligand du récepteur EcR selon l'une quelconque des revendications 1 à 16.
